# EUROPÄISCHE PATENTSCHRIFT

(11) **EP 2 040 636 B1**
(45) Veröffentlichungstag und Bekanntmachung des Hinweises auf die Patenterteilung: **10.09.2014**
(21) Anmeldenummer: 07785596.3
(22) Anmeldetag: 06.07.2007
(51) Int. Cl.: A61B 19/00, G01L 5/16, A61B 17/00, A61M 25/00, A61M 25/09

(54) **KRAFTSENSOR ZUM ERFASSEN EINES KRAFTVEKTORS**
FORCE SENSOR FOR THE DETECTION OF A FORCE VECTOR
CAPTEUR DE FORCE POUR LA DÉTECTION D'UN VECTEUR DE FORCE

(30) Priorität: 06.07.2006 DE 102006031635
(43) Veröffentlichungstag der Anmeldung: 01.04.2009
(73) Patentinhaber: Technische Universität Darmstadt, 64285 Darmstadt (DE)
(72) Erfinder: WERTHSCHÜTZKY, Roland, 14532 Kleinmachnow (DE); MEISS, Thorsten, 64285 Darmstadt (DE); KERN, Thorsten, 64342 Seeheim-Jugenheim (DE); KLAGES, Stephanie, 64807 Dieburg (DE)
(74) Vertreter: Dosterschill, Peter
(86) Internationale Anmeldenummer: PCT/DE2007/001198
(87) Internationale Veröffentlichungsnummer: WO 2008/003307

(56) Entgegenhaltungen:
- EP-A- 0 176 173
- JP-A- 2006 064 465
- US-A- 3 261 204
- US-A- 4 748 858
- US-A- 5 129 265
- US-A- 5 396 887
- US-B1- 6 221 023

## Beschreibung

Die Erfindung betrifft einen Kraftsensor bzw. eine Kraftsensoreinheit, insbesondere für eine lang gestreckte Einrichtung gemäß Oberbegriff des Anspruchs 1.

Ein besonderer Anwendungsfall dieser Erfindung betrifft die Kathetertechnik, die von einer lang gestreckten Einrichtung zum zumindest teilweisen Einführen in einen Organismus durch eine Körperöffnung bestimmt ist. Diese lang gestreckten Einrichtungen kommen vor allem in der minimal invasiven Chirurgie und Diagnostik an insbesondere menschlichen Körpern zum Einsatz. Damit bei der Invasion der lang gestreckten Einrichtung keine Körpergefäße durch die körpernahe Spitze der von dem behandelnden Arzt in der Regel manuell zu bedienenden lang gestreckten Einrichtung verletzt werden, orientiert sich der Arzt notwendigerweise daran, welche Kräfte ihm an einer Handhabe der lang gestreckten Einrichtung mitgeteilt werden. Wegen der Reibung und der eingeschobenen, im Verlauf der Invasion des Katheters oder Führungsdrahtes in den Körper kontinuierlich zunehmenden Katheter-/Führungsdrahtmasse, gibt die dem behandelnden Arzt an der Handhabe mitgeteilte Kraft einen kaum noch nutzbaren Aufschluss über die tatsächlich an der Katheter-/Führungsdrahtspitze auftretenden Kräfte. Damit der behandelnde Arzt der Handhabe des Katheters die richtige Betätigungskraft mitteilen kann, ist ein außergewöhnlich reicher Erfahrungsschatz bei der Bedienung von Kathetern-/Führungsdrähten erforderlich.

Aus der DE 103 03 270 A1 ist eine Katheteranordnung bekannt, bei der die auf die Katheterspitze beim Einschieben wirkende Kraft gemessen wird. Die entsprechende Kraft wird dem Arzt über eine haptische Handhabe taktil mitgeteilt. Auf diese Weise wird das Auffinden beispielsweise von Aderabzweigen oder Perforationen an der Herzscheidewand, gerade für einen unerfahrenen Arzt erleichtert und bildet die Basis für eine intuitive Handhabung der Einrichtung. Eine die Kraft an der Spitze repräsentierende Messgröße verwendende, elektrodynamische Antriebsvorrichtung zur Erzeugung der haptischen Kraftvorspannung ist aus der DE 103 19 081 A1 bekannt. Die hier vorgestellte Erfindung ermöglicht die Kraftmessung an der Spitze der lang gestreckten Einrichtung und die Auswertung der Messsignale, was zur Umsetzung der Erfindung aus der DE 103 03 270 A1 erforderlich ist.

Gemäß der US 6,221,023 B1 wird ein Kraftsensor an der Spitze von Kathetern vorgesehen, der auf einem resistiven Funktionsbetrieb basiert. Die in den Sensor eingeleitete Kraft wird durch eine Widerstandsbrückenschaltung aufgenommen. Der Aufbau dieses Sensors ist aufgrund der großen Teilezahl aufwendig, und die damit verbundenen Fertigungs- und Montagekosten machen den bekannten Sensor insbesondere für Katheter aufgrund deren bevorzugten Einweg-Eigenschaft ungeeignet. Weiterhin ist die Fläche zur Primärkontaktierung des Messelementes senkrecht zur Längsrichtung des Katheters ausgeführt und somit die Fläche zur Kontaktierung durch den Durchmesser der lang gestreckten Einrichtung begrenzt. Deshalb, und wegen der hohen Teileanzahl ist die Miniaturisierbarkeit des Kraftsensors insbesondere unterhalb eines Katheterdurchmessers von weniger als 3 mm nur mit einem äußerst hohen konstruktiven Aufwand realisierbar. Aufgrund der großen Abmessungen ist zu schließen, dass der Sensor den Katheterschlauch an der Spitze vollständig verschließt. Damit ist aber die Funktion des Katheterschlauchs, durch den Instrumente und Flüssigkeiten in den Körper eingebracht werden, nicht mehr gegeben. Eine Integration des Sensors in den bei Katheterisierungen benötigten, wesentlich dünneren Führungsdraht ist aufgrund der großen Abmessungen, der hohen Teileanzahl und der ungünstigen Drahtführung durch die senkrecht zur Längsrichtung orientierten Kontaktfläche nicht möglich.

In der JP 06190050 A wird ein taktiler Sensor beschrieben, der an der Außenwandung von Kathetern angebracht werden kann. Es wird vorgeschlagen, diesen Sensor aus einer sehr dünnen Siliziumscheibe herzustellen. Sehr dünne Elemente können aber die auftretenden Kräfte von ca. 300 mN nicht aufnehmen. Die Verwendung dickerer, stabilerer Siliziumscheiben scheidet aus, da eine Biegung dickerer Scheiben, was zur Integration in die Einrichtung notwendig ist, zum Zerbrechen des Materials führen würde.

In "Beccai L et al.: Silicon-based three axial Force Sensor for Prothetic Applications. Sensors and Microsystems, Proceedings of the 7th Italien Conference 2002" wird ein Kraftsensor vorgestellt, der in Beinprothesen integriert werden soll. Der Kraftvektor kann bestimmt werden, indem die Kraft in Richtung und Amplitude gemessen wird. Zur Messung der Kräfte werden resistive Elemente eingesetzt, die nicht zu einer Wheatstone-Brücke verschaltet sind. Zur Integration in eine lang gestreckte Einrichtung sind aber Brückenverschaltungen anzustreben, wodurch eine hohe Messgenauigkeit erzielt werden kann, da dann die Signale nahezu unabhängig von Zuleitungswiderständen der langen, dünnen Zuleitungen übertragen werden können. Gerade bei in den Körper eingeführten langen Einrichtungen ist sonst das Messsignal abhängig von der Temperatur der Zuleitung und somit von der Einschublänge der Einrichtung. Die Herstellung der Elemente erfordert einen beidseitigen, mehrstufigen Trockenätzprozess. Trotzdem muss dass Messelement noch über einen speziellen Gegenkörper gelagert werden, um eine Auslenkung der Messbalken zu ermöglichen. Der Sensor besteht somit aus mindestens zwei Teilen, die exakt miteinander verbunden sein müssen. Der freigelegte Kraftaufnehmer, genannt Mesa, weist konstruktionsbedingt eine große Länge, nämlich ungefähr eine Länge, die der Dicke des Ausgangsmaterials entspricht, auf. Somit zeigt dieser Sensor prinzipbedingt immer eine um ein Vielfaches höhere Messempfindlichkeit, aber auch Anfälligkeit gegen Zerstörung, gegenüber seitlichen Kräften, im Vergleich zu der in Längsrichtung wirkenden Kraft. Das ist ungünstig, da es wünschenswert ist, besonders die Kräfte in Längsrichtung der Einrichtung zu messen bzw. eine ausgewogene Empfindlichkeit für unterschiedliche Kraftkomponenten zu erzielen. Das Messelement weist eine zur Längsrichtung senkrechte Ebene der elektrischen Kontakte auf, so dass die Kontaktierungsfläche auf den Durchmesser der Einbaufläche begrenzt ist, was eine Kontaktierung erschwert. Das Messelement weist einen gegenüber Führungsdrähten großen Durchmesser von etwa 1 mm auf, so dass eine Integration in den Führungsdraht nicht möglich ist.

Dies zeigt, dass der Stand der Technik von taktilen Sensoren zur Integration in Katheter und Führungsdrähte nicht den gestellten Anforderungen bezüglich Miniaturisierung, hoher Stabilität, einfacher Herstellung und niedriger Kosten entspricht.

Aus US-A-5129265 ist ein Sensor gemäß dem Oberbegriff des Anspruchs 1 zur Messung von zwei Kraftkomponenten bekannt, der einen Materialblock aufweist. Bei der Herstellung sind mehrere, nicht orthogonale Seiten zu bearbeiten. Damit ist diese Herstellung nicht mit den Fertigungsverfahren der Mikrotechnologie zur Fertigung vieler Sensoren zeitgleich parallel im Scheibenverbund kompatibel, die eine Parallelverarbeitung nur von der Vorder- und der Rückseite zulassen.

Aus US-A-3261204 ist ein Kraftsensor mit einem Verformungskörper bekannt, auf den in separaten Arbeitsschritten diskrete Dehnungsstreifen aufgebracht werden. Durch ein derartiges Fertigungsverfahren, das eine Montage von Einzelelementen voraussetzt, sind diese Kraftsensoren in der erzielbaren Miniaturisierbarkeit stark beschränkt.

Der Erfindung liegt die Aufgabe zugrunde, einen Kraftsensor der oben genannten Art anzugeben, der einfach aufgebaut ist, aus einem Teil besteht und hohe Anforderungen an eine Miniaturisierung erfüllt.

Diese Aufgabe wird durch einen Kraftsensor gelöst, der in den Ansprüchen definiert ist.

Mit dem erfindungsgemäßen Kraftsensor wird eine Vielzahl von Vorteilen erzielt.

Der erfindungsgemäße Kraftsensor ist in lang gestreckte Einrichtungen von weniger als 3 mm, vorzugsweise 0,33 mm (1 French) Durchmesser integrierbar; er kann Kräfte erfassen, welche zumindest teilweise an der lang gestreckten Einrichtung in Längsrichtung angreifen, so dass dem Anwender ein kraftabhängiges Drehmoment vermittelt wird, möglichst durch Erfassung des an der Führungsdrahtspitze anliegenden Kraftvektors, also der Bestimmung des Kraftbetrages in drei unabhängigen Richtungen sowie auch der Rückschluss auf die Richtung der angreifenden Kraft möglich ist, so dass Kräfte nach Betrag und Richtung bestimmt werden können.

Der erfindungsgemäße Kraftsensor ist in der Weise ausgelegt, dass eine Kraft erfasst wird, die an einer lang gestreckten Einrichtung, insbesondere einer lang gestreckten medizintechnischen Einrichtung, wie einem Katheter oder Führungsdraht, angreift, welche eine nicht zu vernachlässigende Kraftkomponente in Längsrichtung der lang gestreckten Einrichtung aufweisen kann. Der erfindungsgemäße Sensor hat einen Kraftaufnehmer, an dem zumindest der wesentliche Teil der zu erfassenden Kraft entweder über die lang gestreckte Einrichtung oder direkt in den Sensor gerichtet einleitbar ist.

Der Sensor ist dabei erfindungsgemäß derart beschaffen, dass er an der lang gestreckten Einrichtung anbringbar und insbesondere bei bereits existierenden lang gestreckten Einrichtungen nachrüstbar ist. Die Möglichkeit der einfachen Miniaturisierung der Kraftsensoren erlaubt eine Integration der Kraftsensoren sowohl in die Katheterwandung, ohne die distale Katheteröffnung zu verschließen, als auch die besonders wichtige und anspruchsvolle Integration in den Führungsdraht.

Zur Montage des Sensors an einem Führungsdraht sind Zentrierelemente und Montageanschläge vorgesehen, um die Montage, die bei miniaturisierten Systemen eine große Herausforderung darstellt, einfach und kostengünstig realisieren zu können. Durch besondere Konstruktionsmerkmale lassen sich die Kräfte an der lang gestreckten Einrichtung in Amplitude und darüber hinaus auch in der Wirkrichtung bestimmen.

Der erfindungsgemäße Kraftsensor bietet insbesondere die folgenden Vorteile gegenüber den genannten bekannten Kraftsensoren:
- Mit dem erfindungsgemäßen Sensor besteht die Möglichkeit, eine Kraftsensorik in lang gestreckte Einrichtungen zu integrieren, welche eine laterale Ausdehnung oder einen Durchmesser von weniger als 3 mm, insbesondere 0,33 mm (1 French) aufweisen;
- Der erfindungsgemäße Sensor besteht aus einem einzelnen Teil und ist aus diesem Grund und der Fertigungstechnologie im Scheibenverbund für eine Massenproduktion hervorragend geeignet. Die Fertigungskosten sind sehr gering, Montagekosten für das Sensorelement selbst entfallen, eine Montage an der lang gestreckten Einrichtung ist besonders einfach;
- Durch die spezielle Gestaltung und der monolithischen Integration von Montageelementen ist die Montage der sehr kleinen Kraftsensoren am Führungsdraht einfach möglich;
- Durch die Integration von mechanischen Anschlägen wird eine Zerstörung des Sensors bei Überlast verhindert;
- Die besondere Gestaltung und die damit verbundene hohe mechanische Steifigkeit des Kraftsensors ermöglicht eine sehr einfache, dünne, kostengünstige Gehäusung durch Vergießen oder Umspritzen. Der Kraftsensor erfüllt so ohne weiteres die hohen Anforderungen der Hygiene in der Medizintechnik;
- Die Kopfform des erfindungsgemäßen Sensors kann sehr variabel gestaltet sein, wodurch eine Anpassung an verschiedene Behandlungsszenarien von Katheterisierungen möglich ist;
- Mit dem erfindungsgemäßen Sensor können aufgrund der Nutzung des piezoresistiven Wirkprinzips sehr hohe Auflösungen bei der Kraftbetrags- und Kraftrichtungsmessungen erzielt werden, unter Verwendung einer kostengünstigen externen Auswerteelektronik;
- Mit dem erfindungsgemäßen Sensor können aufgrund der Verwendbarkeit von vorzugsweise Silizium als Grundwerkstoff äußerst genaue Messungen erfolgen, so dass bei Kraftbe- und -entlastung das Ausgangssignal in besonderem Maße die anliegende Kraft wiedergibt;
- Der erfindungsgemäße Sensor kann mit gegenwärtig verfügbarer Technologie noch weit unter die geforderten lateralen Abmessungen von 0,3 mm miniaturisiert werden;
- Auf dem erfindungsgemäßen Sensor ist in einfacher Weise zusätzliche Elektronik monolithisch integrierbar, so dass die Störeinflüsse der Signalübertragung, im besonderen aber auch die Anzahl der notwendigen elektrischen Versorgungsleitungen auf ein Minimum reduziert werden können. Zur Energie- und Signalübertragung können die schon vorhandenen Komponenten der lang gestreckten Einrichtung genutzt werden, wodurch die Kosten der gesamten Einrichtung durch die Integration des Kraftsensors nur sehr gering erhöht werden.

Der erfindungsgemäße Sensor kann Kräfte dem Betrag und/oder der Wirkrichtung nach in Echtzeit und insbesondere kontinuierlich erfassen. Insbesondere ist der erfindungsgemäße Sensor dazu ausgelegt, eine Kraft hauptsächlich in Längsrichtung der lang gestreckten Einrichtung zu erfassen.

Der erfindungsgemäße Sensor ist aus einem Grundelement aufgebaut, welches sich in Abhängigkeit der anliegenden Kraft verbiegt. Diese Verbiegung erzeugt mechanische Spannungen, die auf den gekennzeichneten Messbalken mittels spannungs- und dehnungsempfindlicher Widerstände erfasst werden. Die Änderung dieser Messwiderstände gibt in einem Proportionalverhältnis die anliegende Kraft wieder.

Eine in axialer Richtung eingekoppelte Kraft F_{z} erzeugt in den horizontalen Messbalken nahe der Oberseite Zug-, in der Nähe der Balkenunterseite Druckspannungen. Diese mechanischen Spannungen bewirken in den Balken eindotierten Widerständen gegensinnige Widerstandsänderungen, die beispielsweise über eine Wheatstonsche Brückenverschaltung gemessen werden können. Die Widerstandsänderung, bzw. die Brückenausgangsspannung ist somit eine Maß für die anliegende Kraft F_{z}.

In einem zur axialen Richtung der lang gestreckten Einrichtung ausgerichtetem Messbalken können weitere Widerstände ein- oder aufgebracht gebracht werden. Seitlich an der Kopfstruktur angreifende Kräfte bewirken eine Verbiegung des Balkens, die mechanische Spannungen bewirkt. Diese mechanischen Spannungen können mit Widerständen bestimmt werden kann und stellen ein Maß für die seitlich angreifenden Kräfte dar.

Die auf den Sensor wirkenden Kräfte führen zu Verbiegungen der mechanischen Strukturen. Durch monolithisch integrierte, mechanische Überlastanschläge in dem erfindungsgemäßen Sensor ist es möglich, sehr große Verformungen der Geometrie zu Verhindern und somit einer Zerstörung des Kraftsensors bei zu hoher Last zu vermeiden. Die Integration kann ohne zusätzliche Prozessschritte erfolgen, ein Gegenkörper zur Begrenzung der Verformung ist nicht notwendig.

In den erfindungsgemäßen Sensor werden vorzugsweise Elemente zur Vereinfachung der Montage des Sensors am Führungsdraht integriert. Hierzu ist beispielsweise ein Fuß vorzusehen, der durch eine Verjüngung zum unteren Ende ein leichtes Einführen des Grundkörpers in die Hohlöffnung des Führungsdrahtes ermöglicht. Weiterhin sind Befestigungselemente für weitere Komponenten des Führungsdrahtes, nämlich eines Sicherungsbandes und einer Drahtseele, vorsehbar. Dies ist beispielsweise durch einen Einschnitt am unteren Fußende möglich.

Alle Ausführungsformen können durch Mikrofertigungsverfahren, vorzugsweise die der Volumen-Mikromechanik in Silizium gefertigt werden. Dabei wird eine Siliziumscheibe so strukturiert, dass durch ein Einbringen eines Dotierungsstoffes in das Silizium Widerstände erzeugt werden, die ihren Betrag in Abhängigkeit von der mechanischen Spannung ändern. Diese Widerstände sind aufgrund der elektrischen Wirkung zwischen der Siliziumscheibe und des Dotierstoffes nur an den gewünschten Bereichen elektrisch leitend verbunden, ansonsten untereinander aufgrund der gewählten Herstellung durch Ausbildung eines pn-Überganges voneinander isoliert. Aufgrund der einfacheren Herstellung werden diese Widerstände in der Regel auf der Oberseite des Messelementes und nicht in Vertiefungen in der Siliziumscheibe eingebracht. Die Herstellung der Messelemente kann beispielsweise in folgender Weise erfolgen (Arbeitsfolge ohne Reinigungsschritte):
- Strukturieren der Widerstände
- Öffnen der Kontaktgebiete
- Strukturieren der Leiterbahnen
- Ätzen der mechanischen Struktur bei gleichzeitigem Vereinzeln, z.B. durch Trockenätzen.

Die Herstellung der Elemente weist gegenüber dem Stand der Technik nur wenige Prozessschritte auf. Dies liegt darin begründet, dass zur Vereinzelung der Messelemente kein Sägeprozess erfolgen muss. Weiterhin wird zur Gewährleistung der Verformung des Körpers kein Gegenkörper notwendig, dessen Verbindung mit dem Grundkörper einen weiteren Prozessschritt erforderte. Eine Passivierung der Widerstände kann bei geeigneter Wahl der Prozessparameter schon beim Dotieren erfolgen und stellt somit keinen weiteren Prozessschritt dar. Die Herstellung des Messelementes benötigt insgesamt nur vier Maskenebenen. Die Verfahren der Mikrostrukturierung ermöglichen die Herstellung einer äußerst großen Menge an Sensoren zeitgleich auf einer Scheibe (Batch-Verfahren, Fertigung im Nutzen) und ermöglichen so sehr günstige Messelemente.

Neben der Ausführung als miniaturisiertes piezoresistives Messelement ist es im Allgemeinen auch möglich, die vorgestellten Messelemente makroskopisch aufzubauen und zur Spannungs- bzw. Dehnungsmessung Dehnungsmessstreifen oder Dick- bzw. Dünnschichtwiderstände einzusetzen.

Die Sensoren können beispielsweise an der Spitze von Führungsdrähten, aber auch an beliebiger Stelle in Kathetern eingesetzt werden.

Nachfolgend wird die Erfindung anhand von Ausführungsbeispielen unter Bezug auf die Zeichnung näher erläutert. Es zeigen:
- Fig. 1a:: eine Prinzipskizzenansicht des erfindungsgemäßen Sensors mit symmetrischer, rechteckiger Kopfgestaltung zur Verdeutlichung mathematischer Zusammenhänge;
- Fig. 1b:: einen Ausschnitt aus der Prinzipskizzenansicht 1 a, die Biegewinkel und Biegemomente auf einen vertikalen Balken zur Berechnung der mechanischen Spannungen bei axialer Krafteinleitung durch die Kraft F_{z} verdeutlichend;
- Fig. 2:: eine Ausführung mit abgerundeter Kopfform und nicht senkrechter Gestaltung der Aussparung zur Erzeugung der Balkenstruktur;
- Fig. 3a:: ein Geometriemodell eines bevorzugten Ausführungsbeispiels zur Berechnung der mechanischen Spannungen und elektrischen Widerstandsänderungen mithilfe der Finite-Elemente-Methode (FEM) bei Krafteinleitung aus allen drei Raumrichtungen;
- Fig. 3b:: die durch FEM-Simulation ermittelte Widerstandsänderung für Widerstände, die entlang dem Pfad A und Pfad B angebracht sind, für eine Krafteinleitung von 100 mN in axialer Richtung (F_{z}) in die Sensor-Stirnfläche 103;
- Fig. 4:: ein mögliches Layout für die elektrische Struktur eines Sensors;
- Fig. 5:: eine CAD-Zeichnung eines konstruierten Kraftsensors, der an einem Führungsdraht montiert ist, und
- Fig. 6:: eine Prinzipskizze eines Schnittes nahe der Frontfläche eines Katheters mit integrierten Kraftsensoren.

Aus der Zeichnung ist ersichtlich, daß der erfindungsgemäße Sensor monolithisch aus einem Grundelement (100) aufgebaut ist, welches sich in Abhängigkeit der anliegenden Kraft verbiegt. Diese Verbiegung erzeugt mechanische Spannungen, die auf den Messbalken (101 a und b, 102 a und b) mittels spannungs- oder dehnungsempfindlicher Widerstände erfasst werden. Die Änderung dieser Messwiderstände gibt in einem Proportionalverhältnis die anliegende Kraft wieder.

Eine in axialer Richtung eingekoppelte Kraft F_{z} erzeugt in den horizontalen Messbalken (101 a und b) nahe der Balkenoberseite Zug-, in der Nähe der Balkenunterseite Druckspannungen. Diese mechanischen Spannungen bewirken in den in den Balken eindotierten Widerständen R₁ bis R₄ gleich- bzw. gegensinnige Widerstandsänderungen, die beispielsweise über eine Wheatstonsche Brückenverschaltung gemessen werden können. Die Widerstandsänderung, bzw. die Brückenausgangsspannung ist somit eine Maß für die anliegende Kraft F_{z}.

Vorzugsweise werden die Widerstände zu einer Wheatstonschen Brückenschaltung angeordnet. Dies gewährleistet stabile Messungen. Bei Verschaltung der Widerstände zu einer Wheatstone-Brücke wird das Ausgangssignal für eine Kraftkomponente, beispielsweise bei Einwirkung von F_{z}, maximal, hingegen bei Einwirkung der Kraftkomponente Fy minimal. Beispielsweise durch Vertauschen der Versorgungs- und Signalleitungen in einer Elektronik kann die Empfindlichkeit der Widerstandsverschaltung auf dem Messbalken für eine Kraftkomponente maximiert, für die andere Komponente gleichzeitig minimiert werden. Hierdurch ist die exakte, unabhängige Bestimmung der anliegenden Kraftkomponenten F_{z} und Fy nach Betrag und Richtung möglich. Durch zeitlich schnelles Umschalten der Kraftsensorkontaktierung ist so eine quasi kontinuierliche Bestimmung der Kraftkomponenten F_{z} und Fy möglich.

Zur Messung seitlich angreifender Kräfte Fₓ und Fy ist die Balkenstruktur (106) gut geeignet. Eine an der Frontfläche (105) angreifende Kraft Fₓ führt in Widerständen auf dem Messbalken zu einer Dehnung in Längsrichtung und somit bei in Längsrichtung angeordneten Widerständen zu einer Widerstandszunahme. Obwohl ein einzelner Widerstand zur Bestimmung der Kraftkomponente Fₓ genügt, können bei Nutzung des piezoresistiven Prinzips durch längs- und quer angeordnete Widerstände auf dem Balken (106) gegensinnige Widerstandsänderungen erzielt und somit Spannungsteiler bzw. Brückenschaltungen zur Erhöhung der Messgenauigkeit integriert werden. Eine Kraft Fy bewirkt in dem Balken (106) auf der rechten Seite Zugspannungen, auf der linken Seite Druckspannungen. Diese mechanische Spannung kann durch mindestens einen piezoresistiven Widerstand gemessen werden. Vorzugweise werden zwei nebeneinander vertikal eingebrachte Widerstände verwendet, die zu einem Spannungsteiler verschaltet werden können. Während die Kraft Fy eine gegensinnige Widerstandsänderung in zwei nebeneinander vertikal ausgerichteten Widerständen hervorruft, bewirkt eine Kraft Fₓ eine gleichsinnige Widerstandsänderung beider Widerstände. Sind die Widerstände zu einem Spannungsteiler verschaltet, steigt der Gesamtwiderstand des Spannungsteilers, der somit ein Maß für die Kraft Fₓ ist. Somit ist die Differenz der Widerstandsänderung ein Maß für die Kraft Fy und die Größe der gleichsinnigen Widerstandsänderung ein Maß für die Kraft Fₓ. Aufgrund der kleinen Abmessungen des Kraftsensors ist auch die Verwendung von piezoresistiven Hallelementen sinnvoll. Bei moderatem Miniaturisierungsgrad kann die Verschaltung von mehreren Widerständen zu einer oder mehreren Vollbrücken auf dem Balken (106) die Messgenauigkeit verbessern. Bei sehr hohem Miniaturisierungsgrad kann es sinnvoll sein, nur einen einzelnen Widerstand in der Mitte des Balkens (106) zu integrieren. Dann kann die Verbiegung in x-Richtung über die Zunahme des Einzelwiderstandes, oder bei Reihenschaltung mit weiteren Widerständen über die Änderung des Gesamtwiderstandes bestimmt werden. Eine seitliche Verbiegung in y-Richtung und damit die Kraft Fy kann dann allerdings nicht über diesen Widerstand gemessen werden. Soll die Kraftkomponente Fy trotzdem erfasst werden, so kann dies wie beschrieben über die Widerstandsanordnung auf den Messbalken (101), beispielsweise durch Vertauschen der Versorgungs- und Signalleitungen erfolgen.

Es bestehen vielfältige Möglichkeiten durch unterschiedliche Widerstandsanordnungen mechanische Spannungen zu messen, aus denen die Kräfte Fₓ, Fy, und F_{z} bestimmt werden können. Um möglichst unabhängige Funktionen der Widerstandsänderung von den einzelnen Kraftkomponenten zu erhalten, sind weitere Widerstandsanordnungen denkbar, beispielsweise die Aufnahme von horizontal orientierten Widerständen und die Anordnung zu Spannungsteilern oder Brückenschaltungen oder piezoresistiven Hallelementen. Auch ergeben sich in der vertikalen Balkenstruktur (102) sowie in der Kopfstruktur (103) mechanische Druck- und Zugspannungen, die für die Messung der Kräfte über Widerstände geeignet sind. Dies bietet bei einem hohen Miniaturisierungsgrad die vorteilhafte Möglichkeit, eine Vollbrückenstruktur zu realisieren, ohne Widerstände mit der notwendigen gegenläufigen Widerstandanordnung direkt nebeneinander anordnen zu müssen. So kann der verbleibende Platz auf einem Messbalken für elektrische Leiterbahnen verwendet werden. Es ist in dieser Art auch möglich, die Dicke des Messbalkens (101) weiter zu verringern und so noch kleinere Kräfte messbar zu machen.

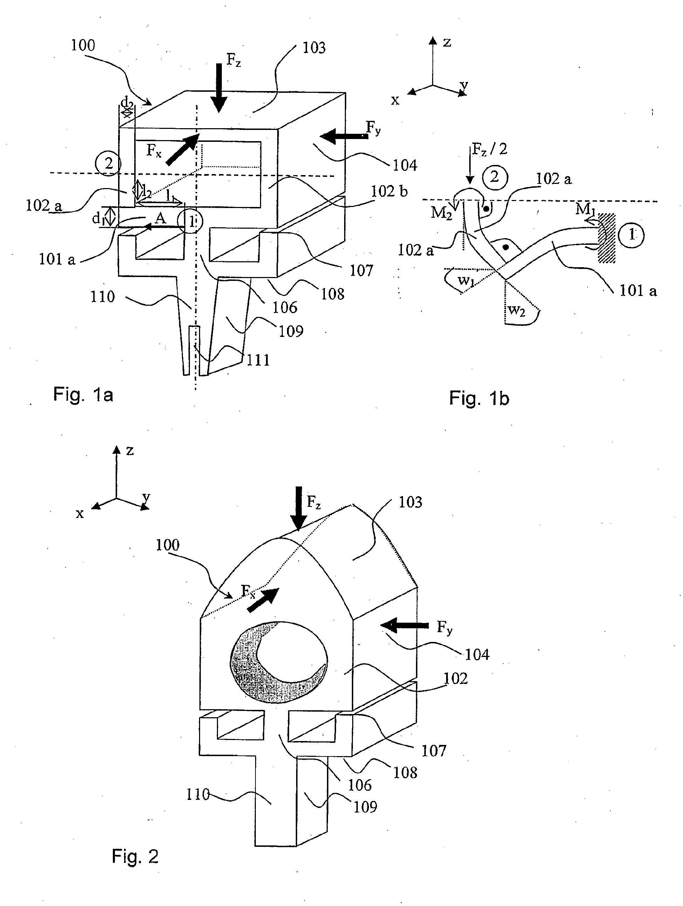

Weiterhin ist es bei dem erfindungsgemäßen Sensor in vielfältiger Weise einfach möglich, durch einen mechanischen Anschlag (107) die Auslenkung des Balkens (101) und die Verkippung des Balkens (106) zu begrenzen. Es kann somit ein Überlastschutz realisiert werden. Dieser Überlastschutz kann wie Fig. 1 a durch einen einfachen Anschlag realisiert werden. Auch alternative Anordnungen, beispielsweise eine Integration zwischen Stirnfläche (103) und Messbalken (106) ist vorstellbar. Der Entwurf des Überlastschutzes ist den möglichen Spaltbreiten und dem möglichen Aspektverhältnis des Herstellungsprozesses anzupassen.

Die Fig. 1a zeigt weiterhin einen sich zum unteren Ende des Grundkörpers verjüngenden Fußbereich. Dies vereinfacht die Montage des Kraftsensors am Führungsdraht.

Die Fig. 1b zeigt einen Ausschnitt des Messbalkens (101 a). Es wird gezeigt, dass sich bei Einkopplung einer Kraft F_{z} in den Kraftsensor eine Verbiegung des Messbalken (101 a) einstellt. Diese Verbiegung bewirkt wiederum eine Verbiegung des linken vertikalen Messbalkens (102a), wodurch ein im Messbalken (101 a) wirkendes Moment M₁ verringert wird und der Messeffekt sinkt. Für eine Sensoroptimierung ist deshalb auch die Betrachtung der gesamten Kopfstruktur des Kraftsensors notwendig.

In Fig. 2 wird eine weitere Ausführungsform des erfindungsgemäßen Kraftsensors gezeigt. Hier ist die Stirnfläche (103) abgerundet ausgeführt. Die Abrundung kann vorteilhaft sein, um das distale Ende der lang gestreckten Einrichtung möglichst so zu gestalten, dass im Gebrauch keine Verletzungen des Patienten entstehen. Allerdings kann auch eine Gestaltung als Spitze oder Schneide erfolgen, um beispielsweise Verengungen, Verschlüsse oder Gewebe besser zu durchdringen oder Kräfte reproduzierbarer in den Kraftsensor einzukoppeln. Weiterhin ist in Fig. 2 die Balkenstruktur durch Einbringen eines Loches erzeugt.

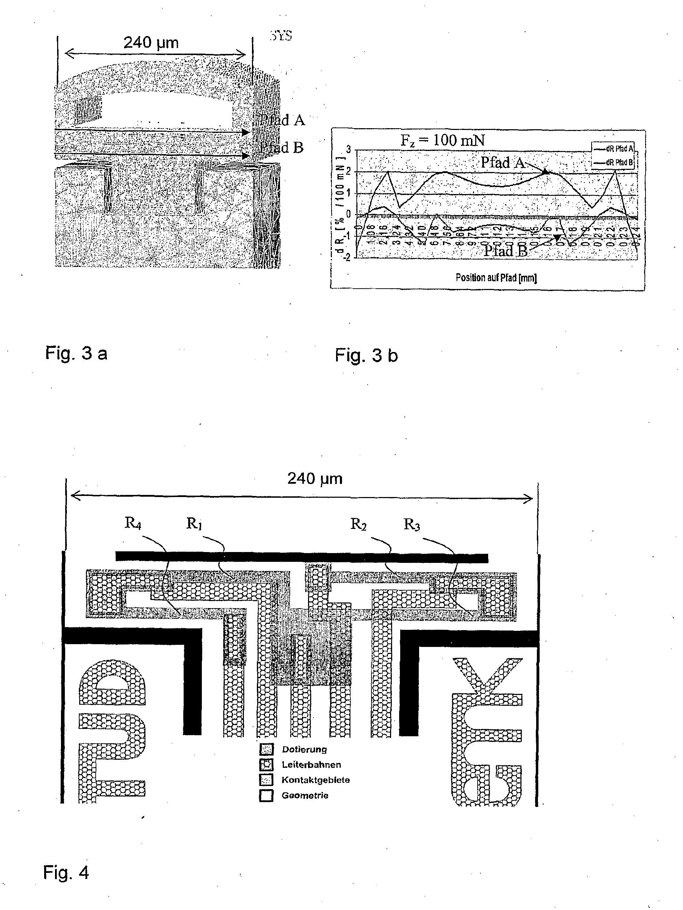

In Fig. 3a ist ein Geometriemodell eines bevorzugten erfindungsgemäßen Kraftsensors zur Berechnung der mechanischen Spannungen und elektrischen Widerstandsänderungen mithilfe der Finite-Elemente-Methode (FEM) bei Krafteinleitung aus allen drei Raumrichtungen aufgezeigt. Auf Pfad A und Pfad B werden die mechanischen Spannungen berechnet und über mathematische Beschreibungen des piezoresistiven Effektes die Widerstandsänderungen der Widerstände berechnet. In Fig. 3b ist die Widerstandsänderung aufgezeigt und es lassen sich effektive Widerstandsänderung von 0,5% bis 1,5% bei Nennlast von F_{z} = 100 mN erwarten.

In Fig. 4 ist beispielhaft ein mögliches Layout für die elektrische Struktur eines erfindungsgemäßen Sensors aufgezeigt. Hier sind nur in dem vertikalen Messbalken (101 a und b) Widerstände eindotiert, die als Wheatstone-Brücke verschaltet sind.

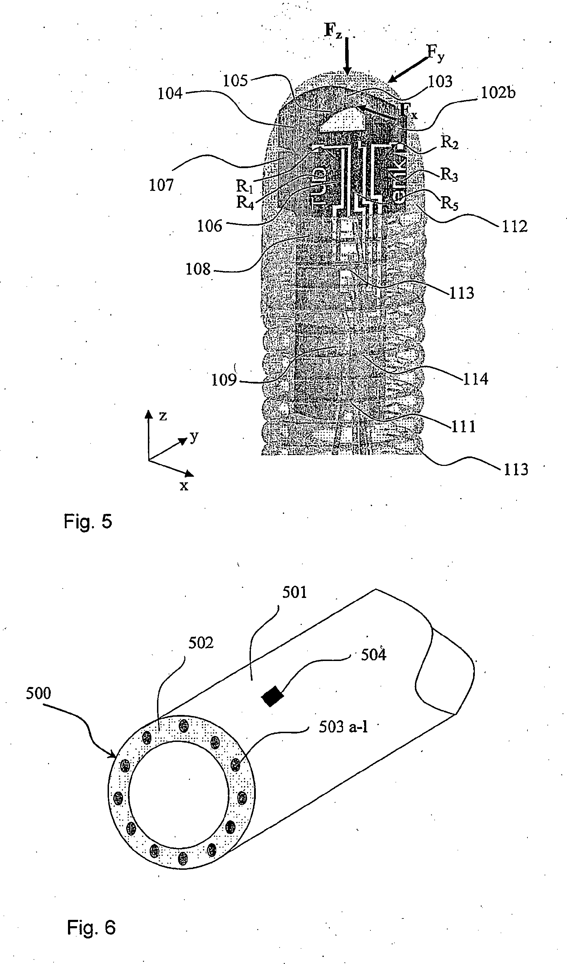

In Fig. 5 ist ein in die Spitze eines Führungsdrahtes integrierter, elektrisch kontaktierter Sensor dargestellt. Die Gehäusung erfolgt hier durch einen Verguss mit einem relativ weichen, biokompatiblen Material, beispielsweise Silikon oder Polyurethan. Die hohe Steifigkeit des Messelementes im Vergleich zum Gehäusungswerkstoff gewährleistet einen geringen Einfluss der Gehäusung auf das Messsignal. Durch die halbtransparente Darstellung sind die Kontaktflächen und Anschlussleitungen erkennbar.

Wie in Fig. 6 gezeigt, ist es auch möglich, die Kraftsensoren aufgrund ihrer kleinen Abmessungen an beliebigen Stellen innerhalb oder an Katheterschläuchen zu integrieren. Auch können in eine Katheterwandung mehrere Kraftsensoren integriert werden. Dies ermöglicht örtlich verteilte Kraftprofile aufzunehmen. Durch die hohe Miniaturisierung der Sensoren bleibt die distale Öffnung des Katheters voll erhalten. Die erfindungsgemäßen Kraftsensoren sind vorzugsweise am distalen Ende der lang gestreckten Einrichtung ein- oder angebracht, oder aber an einer beliebigen Stelle an oder in der lang gestreckten Einrichtung.

Es ist auch möglich, eine Auswerteelektronik direkt auf oder an dem Messelement unterzubringen. Vorteile lassen sich beispielsweise erzielen, indem durch eine Vorverstärkung eine erhöhte Signalspannung erzeugt wird. Dadurch steigt das Signal- zu Rauschleistungsverhältnis (SNR) und somit auch die Auflösung. Durch codierte oder modulierte Übertragung der Signale oder durch spannungsproportionale Stromwandlung können bei integrierter Elektronik die Signale mit geringerem Einfluss der Zuleitung übertragen werden. Durch die Integration von Konstantspannungsquellen kann eine Konstantspannungsspeisung realisiert werden, so dass die Auflösung für seitliche Kraftkomponenten erhöht wird, ohne dass der Leitungswiderstand einen merkenswerten Einfluss auf das Messergebnis ausübt. Weiterhin ist es durch eine integrierte Elektronik möglich, elektrische Differenzspannungen an den Widerständen direkt zu messen und somit die Richtung und den Betrag von Kräften sehr genau zu bestimmen. Durch eine integrierte Elektronik kann eine Potentialanpassung der Signale auf dem Chip erfolgen und elektrische Differenzspannungen auf eine einzelne Masseleitung bezogen werden. Hierdurch können elektrische Leitungen eingespart werden. Dadurch sinkt der Montageaufwand und somit die Herstellungskosten der gesamten Einrichtung. Der Platzbedarf der Leitungen wird weiterhin verringert, wodurch eine weitere Miniaturisierung des Durchmessers der lang gestreckten Einrichtung möglich wird. Durch die Modulation der Signale in einer im Messelement integrierten Primärelektronik ist es möglich, die Leitungszahl auf zwei, und im Speziellen auf eine Leitung zu reduzieren. Durch Integration einer drahtlosen Sendeeinheit ist auch eine drahtlose Übertragung von Energie und Signal möglich.

Die in der vorstehenden Beschreibung, in den Zeichnungen sowie in den Ansprüchen offenbarten Merkmale der Erfindung können sowohl einzeln als auch in beliebigen Kombinationen für die Verwirklichung der Erfindung in ihren verschiedenen Ausführungsformen wesentlich sein.

### Bezugszeichentiste

- 100: Grundkörper
- 101a: linker horizontaler Messbalken
- 101b: rechter horizontaler Messbalken
- 102a: linker vertikaler Messbalken
- 102b: rechter vertikaler Messbalken
- 103: Stirnfläche, Krafteinleitung aus axialer Richtung (F_{z})
- 104: linke Fläche, Krafteinleitung aus y-Richtung (Fy)
- 105: Frontfläche, Krafteinleitung aus x-Richtung (Fₓ)
- 106: vertikaler Messbalken zur Messung radial eingeleiteter Kräfte (Fₓ, Fy)
- 107: mechanischer Anschlag als Überlastschutz
- 108: mechanischer Anschlag als Montagehilfe
- 109: Balken zur Unterbringung von Kontaktflächen, zur Befestigung des Messelementes und zur Zentrierung im Führungsdraht bei der Montage
- 110: Fläche für Bondkontakte
- 111: Einschnitt zur Befestigung einer Führungsdrahtseele und eines Sicherungsbandes
- 112: Gehäusung
- 113: Führungsdrahtwendel
- 114: Energie-und Signalleitungen
- R₁..R₅: Elektrische Widerstände, die aufgrund mechanischer Dehnung und Spannung ihren Grundwiderstand verändern
- F_{z}: Kraftkomponente aus z-Richtung, axial, in Längsrichtung auf den Draht wirkende Kraftkomponente
- Fₓ: Kraftkomponente aus x-Richtung
- Fy: Kraftkomponente aus y-Richtung
- F: Kraftvektor, beinhaltet die Kraftkomponeten F_{z}, Fₓ und Fy
- 500: Katheterschlauch mit integrierten Kraftsensoren
- 501: Außenhülle des Katheters
- 502: Vorderes Ende des Katheters
- 503: Kraftsensoren zur Aufnahme der Kräfte an der Katheterspitze
- 504: Kraftsensoren zur Aufnahme der Kräfte an einem beliebigen Ort auf / in dem Katheter

## Patentansprüche

1. Kraftsensor zum Erfassen mindestens eines Kraftvektors, insbesondere eines auf eine lang gestreckte Einrichtung, insbesondere eine lang gestreckte medlzintechnische Einrichtung, wie einen Katheter oder Führungsdraht, wirkenden Kraftvektors (F) mit einer nicht zu vernachlässigenden Kraftkomponente in Längsrichtung der lang gestreckten Einrichtung, bestehend aus einem monolithischen Grundkörper (100) und aus mindestens einem spannungs- und/oder dehnungsempfindlichen Widerstand (R₁,..., R₆),
**dadurch gekennzeichnet, dass**
- der Kraftsensor in Silizium gefertigt ist, aus dem monolithischen Grundkörper (100) aus einem einzelnen Teil hergestellt werden kann und zur Realisierung einer Messfunktion kein Gegenkörper verwendet werden muss,
- eine Energie- und Signalleitung zu einem Messbereich für eine Kraftkomponente (F_{z}) über einen axial zu der lang gestreckten Einrichtung ausgerichteten Bereich (102) erfolgt, der schmaler als die maximale Breite einer Kopffläche (103) des Kraftsensors gestaltet ist;
- elektrische Anschlussflächen (110) in einer nicht senkrechten Weise zur axialen Richtung der lang gestreckten Einrichtung angebracht sind,
- der monolithische Grundkörper (100) einen Anschlussbereich (109) aufweist, der mechanische Montageschrägen und Montageanschläge (108) bereitstellt, die eine exakte Positionierung und Befestigung in einem Hohlkörper vereinfachen,
- dass mindestens ein Widerstand (R1, ..., R6) in Messbalken (101, 102, 106) des monolithischen Grundkörpers (100) eindotiert ist,
- dass die Messbalken durch Einschnitte begrenzt sind, deren Kanten mit der Oberfläche nahezu senkrechte Winkel bilden, und
- der monolithische Grundkörper (100) mindestens einen monolithisch integrierten mechanischen Anschlag (107, 108) aufweist.

2. Kraftsensor nach Anspruch 1, **dadurch gekennzeichnet, dass** mindestens ein Widerstand (R1, ..., R6) in der Mitte eines vertikalen Messbalkens (101, 102) des monolithischen Grundkörpers (100) angeordnet ist.

3. Kraftsensor nach Anspruch 1, **dadurch gekennzeichnet, dass** zwei Widerstände (R1, ..., R6) gegenüberliegend, jeweils einer in der Nähe einer Außenkante eines vertikalen Messbalkens (102) des monolithischen Grundkörpers (100), angeordnet sind.

4. Kraftsensor nach einem der vorstehenden Ansprüche, **dadurch gekennzeichnet, dass** Widerstände (R1, ..., R6) wenigstens teilweise zu Wheatstonschen-Brückenschaltungen verschaltet sind, und dass die Brückenschaltungen offen oder geschlossen ausgeführt sind.

5. Kraftsensor nach einem der vorstehenden Ansprüche, **dadurch gekennzeichnet, dass** mindestens ein Widerstand (R1, ..., R6) für jede zu messende Kraftkomponente in den monolithischen Grundkörper (100) integriert ist.

6. Kraftsensor nach einem der vorstehenden Ansprüche, **dadurch gekennzeichnet, dass** eine Elektronik zur Signalverarbeitung, zur Energiespeisung und/ oder zur Datenübertragung integriert ist.

7. Kraftsensor nach einem der vorstehenden Ansprüche, **dadurch gekennzeichnet, dass** der Kraftsensor in eine lang gestreckte Einrichtung von weniger als 3 mm Durchmesser integrierbar ist.

8. Kraftsensor nach einem der vorstehenden Ansprüche, dadurch gekennzeich-net, dass der Kraftsensor in die Spitze eines Führungsdrahtes integrierbar ist, und dass eine Gehäusung aus einem biokompatiblen Material besteht.

9. Kraftsensor nach einem der Ansprüche 1 bis 7, **dadurch gekennzeichnet, dass** 9. der Kraftsensor innerhalb oder an Katheterschläuchen (500) integrierbar ist.

10. Kraftsensor nach einem der vorstehenden Ansprüche, **dadurch gekennzeichnet, dass** die Kopffläche (103) des Kraftsensors plan oder rund ausgestaltet ist oder mit einer Spitze oder Schneide versehen ist.

11. Kraftsensor nach einem der vorstehenden Ansprüche, **dadurch gekennzeichnet, dass** der Kraftsensor in der Weise ausgestaltet ist, dass er neben einer in Längsrichtung der lang gestreckten Einrichtung wirkenden Kraft (F_{z}) auch mindestens eine seitliche angreifende Kraftkomponente (Fₓ, Fy) misst.

12. Kraftsensor nach einem der vorstehenden Ansprüche, **dadurch gekennzeichnet, dass** der Kraftsensor in der Weise ausgestaltet ist, dass mechanische Spannungen und elektrische Widerstandsänderungen nach der Finale-Elemente-Methode berechnet werden.

## Claims

1. Force sensor for the detection of at least one force vector, in particular of a force vector (F) acting on an elongate device, in particular an elongate medical-technical device such as a catheter or guide wire, said force vector having a not inconsiderable force component in the longitudinal direction of the elongate device consisting of a monolithic basic body (100) and of at least one resistor (R₁, ..., R₆) which is sensitive to tension and/or expansion, **characterised in that**
- the force sensor is manufactured in silicon, can be produced from the monolithic basic body (100) from a single part, and no counterpart body needs to be used to carry out a measuring function,
- power and signal conduction to a measuring region for a force component (F_{z}) is effected via a region (102) oriented axially with respect to the elongate device, the region being narrower than the maximum width of a head surface (103) of the force sensor;
- electric connection surfaces (110) are mounted in a non-perpendicular manner with respect to the axial direction of the elongate device,
- the monolithic basic body (100) has a connection region (109) which provides mechanical mounting slopes and mounting stops (108) which simplify precise positioning and attachment in a hollow body,
- at least one resistor (R₁, ..., R₆) is doped into measuring bars (101, 102, 106) of the monolithic basic body (100),
- the measuring bars are defined by notches, the edges of which form an almost perpendicular angle with the surface, and
- the monolithic basic body (100) has at least one monolithically integrated mechanical stop (107, 108).

2. Force sensor as claimed in claim 1, **characterised in that** at least one resistor (R₁, ..., R₆) is disposed in the middle of a vertical measuring bar (101, 102) of the monolithic basic body (100).

3. Force sensor as claimed in claim 1, **characterised in that** the two resistors (R₁, ..., R₆) are oppositely disposed, each in the proximity of an outer edge of a vertical measuring bar (102) of the monolithic basic body (100).

4. Force sensor as claimed in any one of the preceding claims, **characterised in that** the resistors (R₁, ..., R₆) are at least partially connected to form Wheatstone bridge circuits and that the bride circuits are formed open or closed.

5. Force sensor as claimed in any one of the preceding claims, **characterised in that** at least one resistor (R₁, .... R₆) is integrated into the monolithic basic body (100) for each force component to be measured.

6. Force sensor as claimed in any one of the preceding claims, **characterised in that** electronics for signal processing, power storage and/or data transmission are integrated.

7. Force sensor as claimed in any one of the preceding claims, **characterised in that** the force sensor can be integrated into an elongate device of less than 3 mm diameter.

8. Force sensor as claimed in any one of the preceding claims, **characterised in that** the force sensor can be integrated into the tip of a guide wire and that a housing consists of a bio-compatible material.

9. Force sensor as claimed in any one of claims 1 to 7, **characterised in that** the force sensor can be integrated within or on catheter tubes (500).

10. Force sensor as claimed in any one of the preceding claims, **characterised in that** the head surface (103) of the force sensor is formed planar or round or is provided with a tip or cutting edge.

11. Force sensor as claimed in any one of the preceding claims, **characterised in that** the force sensor is formed in such a way that, in addition to a force (F_{z}) acting in the longitudinal direction of the elongate device, it also measures at least one laterally impinging force component (Fₓ, F_{y}).

12. Force sensor as claimed in any one of the preceding claims, **characterised in that** the force sensor is formed in such a way that mechanical stresses and changes in electrical resistance are calculated according to the finite element method.

## Revendications

1. Capteur de force pour la détection d'au moins un vecteur de force, en particulier d'un vecteur de force (F) agissant sur un dispositif allongé, en particulier un dispositif technique médical allongé, comme un cathéter ou un fil de guidage, avec une composante de force non négligeable dans la direction longitudinale du dispositif allongé, constitué d'un corps de base monolithique (100) et d'au moins une résistance sensible à la tension et/ou à l'extension (R₁, ..., R₆), **caractérisé par le fait que**
- le capteur de force est fabriqué en silicium, qu'il peut être confectionné à partir du corps de base monolithique (100) en une pièce unique et qu'aucun corps antagoniste ne doit être utilisé pour la réalisation d'une fonction de mesure,
- une ligne d'énergie et de signaux vers une zone de mesure pour une composante de force (F_{z}) se réalise via une zone (102) orientée axialement par rapport au dispositif allongé, qui est conçue plus étroite que la largeur maximale d'une face de tête (103) du capteur de force,
- des surfaces de raccordement électrique (110) sont apposées d'une manière non perpendiculaire à la direction axiale du dispositif allongé,
- le corps de base monolithique (100) présente une zone de raccordement (109), qui fournit des chanfreins de montage et des butées de montage (108), qui facilitent un positionnement exact et une fixation dans un corps creux,
- au moins une résistance (R₁, ..., R₆) est dopée dans des colonnes de mesure (101, 102) du corps de base monolithique (100),
- les colonnes de mesure sont délimitées par des entailles dont les arêtes forment avec la surface un angle pratiquement perpendiculaire, et
- le corps de base monolithique (100) présente au moins une butée mécanique monolithiquement intégrée (107, 108).

2. Capteur de force selon la revendication 1, **caractérisé par le fait qu'**au moins une résistance (R₁, ..., R₆) est disposée au milieu d'une colonne de mesure verticale (101, 102) du corps de base monolithique (100).

3. Capteur de force selon la revendication 1, **caractérisé par le fait que** deux résistances (R₁, ..., R₆) sont disposées en regard, chacune à proximité d'une arête externe d'une colonne de mesure verticale (102) du corps de base monolithique (100).

4. Capteur de force selon l'une des revendications précédentes, **caractérisé par le fait que** des résistances (R₁, .... R₆) sont raccordées au moins partiellement à des circuits à pont de Wheatstone, et que les circuits à pont sont réalisés sous la forme ouverte ou fermée.

5. Capteur de force selon l'une des revendications précédentes, **caractérisé par le fait qu'**au moins une résistance (R₁, .... R₆) est intégrée dans le corps de base monolithique (100) pour chaque composante de force devant être mesurée.

6. Capteur de force selon l'une des revendications précédentes, **caractérisé par le fait qu'**un système électronique est intégré pour le traitement des signaux, pour l'alimentation électrique et/ou pour la transmission de données.

7. Capteur de force selon l'une des revendications précédentes, **caractérisé par le fait que** le capteur de force peut être intégré dans un dispositif allongé ayant un diamètre de moins de 3 mm.

8. Capteur de force selon l'une des revendications précédentes, **caractérisé par le fait que** le capteur de force peut être intégré dans la pointe d'un fil de guidage, et qu'une enceinte est constituée d'un matériau biocompatible.

9. Capteur de force selon l'une des revendications 1 à 7, **caractérisé par le fait que** le capteur de force peut être intégré à l'intérieur ou sur des tubes de cathéter (500).

10. Capteur de force selon l'une des revendications précédentes, **caractérisé par le fait que** la face de tête (103) du capteur de force est aménagée de manière plane ou ronde ou est pourvue d'une pointe ou d'une lame.

11. Capteur de force selon l'une des revendications précédentes, **caractérisé par le fait que** le capteur de force est aménagé de telle sorte qu'il mesure, outre une force (F_{z}) agissant dans la direction longitudinale du dispositif allongé, également au moins une composante de force venant en prise latéralement (Fₓ, F_{y}).

12. Capteur de force selon l'une des revendications précédentes, **caractérisé par le fait que** le capteur de force est aménagé de telle sorte que des tensions mécaniques et des variations de tension électrique sont calculées d'après la méthode des éléments finis.
